Europäisches Patentamt

European Patent Office

Office européen des brevets

(11) Publication number: **0 200 966 B1**

# EUROPEAN PATENT SPECIFICATION

(45) Date of publication of patent specification: **21.07.93** (51) Int. Cl.⁵: **A61K 37/465**

(21) Application number: **86105235.5**

(22) Date of filing: **16.04.86**

(54) **Method of stabilizing urokinase precursor and dry preparation containing said precursor.**

(30) Priority: **16.04.85 JP 79428/85**
**16.04.85 JP 79429/85**

(43) Date of publication of application:
**12.11.86 Bulletin 86/46**

(45) Publication of the grant of the patent:
**21.07.93 Bulletin 93/29**

(84) Designated Contracting States:
**BE CH DE FR GB LI NL SE**

(56) References cited:
**EP-A- 0 139 447**
**EP-A- 0 151 996**
**EP-A- 0 190 041**
**GB-A- 2 021 412**
**US-A- 4 245 051**

**PATENT ABSTRACTS OF JAPAN, vol. 8, no. 260 (C-254)[1697], 29th November 1984; & JP-A-59 139 323 (MIDORI JUJI K.K.) 10-08-1984**

**PATENT ABSTRACTS OF JAPAN, vol. 8, no. 71 (C-217)[1508], 3rd April 1984; & JP-A-58 224 687 (TOUBISHI YAKUHIN KOGYO K.K.) 27-12-1983**

(73) Proprietor: **GREEN CROSS CORPORATION**
**3-3, Imabashi 1-chome Chuo-ku**
**Osaka-shi Osaka(JP)**

(72) Inventor: **Morimoto, Kazuo**
**142, Oikezaka-cho**
**Fukuchiyama-shi Kyoto(JP)**
Inventor: **Narita, Shusaku**
**6, Higashi Hirano-cho**
**Fukuchiyama-shi Kyoto(JP)**
Inventor: **Kondo, Masahide**
**6, Higashi Hirano-cho**
**Fukuchiyama-shi Kyoto(JP)**
Inventor: **Ishikawa, Syoichi**
**6, Higashi Hirano-cho**
**Fukuchiyama-shi Kyoto(JP)**
Inventor: **Ohara, Kazuhiro**
**6, Higashi Hirano-cho**
**Fukuchiyama-shi Kyoto(JP)**

(74) Representative: **Eitle, Werner, Dipl.-Ing. et al**
**Hoffmann, Eitle & Partner Patentanwälte Arabellastrasse 4**
**W-8000 München 81 (DE)**

## Description

The present invention relates to a stabilized urokinase precursor dry preparation.

BACKGROUND OF THE INVENTION

A urokinase precursor secreted from human kidney cells does not exhibit urokinase activity by itself. However, when digested with a proteinase, such as plasmin, the precursor is transformed into a molecule which shows a high urokinase activity.

The urokinase precursor has a high affinity to fibrin and reaches the fibrin constituting a blood clot without acting on or decomposing fibrinogen in the blood plasma. Thereafter, the precursor exhibits the urokinase activity by the action of plasmin. That is, the urokinase precursor causes limited fibrin dissolution in the blood clot site and dissolves the blood clot selectively and efficiently. Therefore, the precursor is believed to be useful as a novel medicine for treatment of the disease of thrombosis.

The urokinase precursor is unstable in a highly purified condition, or dried or freeze dried condition, although it is relatively stable when stored in a neutral solvent in a high concentration during the processes of preliminary purification and final purification.

SUMMARY OF THE INVENTION

An object of the present invention is to provide a dry preparation containing a stabilized urokinase precursor.

It has been found that a dried urokinase precursor is stabilized when a mixture of (a) at least one member of inorganic salts, organic acid salts, and polar amino acids and their salts, and (b) human albumin is added to the precursor.

The present invention relates to a dry preparation containing a stabilized urokinase precursor.

DETAILED DESCRIPTION OF THE INVENTION

The urokinase precursor as used herein includes those precursors obtained from urine, human kidney cells, and by the genetic engineering (see JP-A-62981/1985 corresponding to U.S. Patent Application Serial No. 06/648,134, for example).

The effect of the present invention is particularly remarkable in a highly purified urokinase precursor, such as a urokinase precursor of a concentration that the specific activity is 100,000 IU/mg protein. The symbol "IU" is an abbreviation of an international unit of UK. 1 IU/ml, which is equivalent to the UK activity when 1 ml of the precursor is treated with plasmin, means that 1 ml of the precursor, when treated with plasmin, has the same activity as that of 1 IU of UK.

Examples of the inorganic salts that are used in the present invention are sodium chloride and sodium citrate.

Examples of the polar amino acids that are used in the present invention is arginine.

Suitable examples of the salts of the above polar amino acids is arginine chloride.

Among the above substances which can be used as component (a), organic acid salts and poler amino acids, more particularly arginine and sodium citrate, are preferred.

The albumin as used herein is preferably albumin obtained from human in view of the problem of antigenicity. There are no special limitations to the albumin as long as it is purified for the purpose of use for medical treatment. The purity is preferred to be such that the albumin content as determined by electrophoresis is not less than 80%. Methods of obtaining human albumin include the ethanol fractionation method (see Japanese Patent Publication Nos. 2869/72 and 5297/60), and the method comprising heating in the presence of organic acids (see Japanese Patent Publication Nos. 1604/68 and 40132/76). It is particularly preferred to subject the albumin to heat treatment (preferably at 60°C for about 10 hours) for inactivation of hepatitis virus, for example.

Albumin is added in an amount corresponding to 10 to 60 mg, preferably 25 to 50 mg, and the inorganic salt, organic acid salt, or polar amino acid or its salt is added in an amount corresponding to 2 to 50 mg, preferably 5 to 30 mg, per 10,000 to 250,000 IU of the urokinase precursor.

The stabilizers i.e., inorganic salts, organic acid salts, polar amino acids or their salts, are added with good results at any stage during the process that the urokinase precursor is placed under conditions where the precursor could be inactivated, such as the purification and storage of the urokinase precursor.

The present invention is described in greater detail with reference to the following examples.

2

The activity of the urokinase precursor was measured, after activation, with plasmin, by the use of a synthetic substrate (Glt-Gly-Arg-MCA).

EXPERIMENT 1

To a solution of a purified urokinase precursor (specific activity: 135,000 IU/mg protein) in a 0.10 M phosphate buffer (pH:6.0) as a solvent was added a solution of albumin in the same buffer as above as a solvent to prepare 25,000 IU/ml of an aqueous urokinase precursor solution containing 20 mg/ml of human albumin. Various additives (inorganic salts, organic acid salts, or polar amino acids or their salts) were added to the above prepared aqueous urokinase precursor solution so that the total amount of the additives added was 8 mg/ml and then freeze dried. For comparison, the aqueous urokinase precursor solution not containing any additive, and the aqueous urokinase precursor solution with only human albumin added thereto were freeze dried in the same manner as above.

These compositions were measured for the remaining urokinase precursor titer (%) just after its freeze drying and after storage at 50°C for 3 months. The results are shown in Table 1.

Table 1

| Run No. | Human Albumin (mg/ml) | Additive (Total Amount) | Residual Titer (%) | |
|---|---|---|---|---|
| | | | Just after Freeze Drying | After Storage at 50°C for 3 Months |
| 1 | 20 | Sodium citrate (8 mg/ml) | 99.5 | 99.8 |
| 2 | " | Sodium mandelate (8 mg/ml) | 99.0 | 96.1 |
| 3 | " | Sodium phosophate (8 mg/ml) | 96.5 | 86.5 |
| 4 | " | NaCl (Table salt) (8 mg/ml) | 98.6 | 95.2 |
| 5 | " | Potassium chloride (8 mg/ml) | 96.5 | 86.2 |
| 6 | " | Aspartic acid (6.4 mg/ml) | 97.5 | 93.8 |
| 7 | " | Glutamic acid (6.4 mg/ml) | 97.2 | 94.2 |
| 8 | " | Sodium glutamate (6.4 mg/ml) | 97.6 | 95.8 |
| 9 | " | Arginine (6.4 mg/ml) | 99.5 | 98.8 |
| 10 | " | Lysine (6.4 mg/ml) | 97.0 | 95.7 |
| 11 | " | Histidine (6.4 mg/ml) | 96.5 | 88.6 |
| 12 | " | - | 96.7 | 60.2 |
| 13 | - | - | 94.0 | 2.5 |
| Note: Run Nos. 1 - 11: Examples of the present invention | | | | |
| Run Nos. 12 and 13: Control | | | | |

EXPERIMENT 2

Freeze dried preparations were prepared in the same manner as in Experiment 1 containing human albumin, sodium citrate, salt (NaCl), arginine, or sodium glutamate which rates were varied.

The preparations were measured for the residual urokinase precursor titer (%) just after preparation and after storage at 50°C for 1 month. The results are shown in Table 2.

Table 2

| Run No. | Human Albumin | Formation Additive Type | Amount (mg/ml) | Residual Titer (%) Just After Freeze Drying | After Storage at 50°C for 1 Month |
|---|---|---|---|---|---|
| 1 | 20 mg/ml | Sodium citrate | – | 77.1 | 75.2 |
| 2 | " | do | 3.2 | 87.3 | 86.4 |
| 3 | " | do | 6.4 | 99.3 | 99.2 |
| 4 | " | Salt | – | 76.3 | 75.2 |
| 5 | " | do | 3.2 | 84.2 | 84.1 |
| 6 | " | do | 6.4 | 97.8 | 97.4 |
| 7 | " | Arginine | – | 94.0 | 75.2 |
| 8 | " | do | 3.2 | 99.5 | 86.4 |
| 9 | " | do | 6.4 | 99.5 | 99.2 |
| 10 | " | Sodium glutamate | – | 94.0 | 75.2 |
| 11 | " | do | 3.2 | 97.0 | 84.1 |
| 12 | " | do | 6.4 | 97.6 | 97.4 |

The titer shown in Tables 1 and 2 is a residual activity against the titer before the above treatment as 100.

EXAMPLE 1

25,000 IU of a purified urokinase precursor (specific activity: 135,000 IU/mg protein), 20 mg of human albumin, and 6.4 mg of sodium citrate were dissolved in 1 ml of a 0.1 M phosphate buffer (pH, 7.0), aseptically filtered, charged in a vial bottle, and then freeze dried to prepare an injection preparation containing 25,000 units of urokinase precursor, 20 mg of human albumin and 6.4 mg of sodium citrate.

EXAMPLE 2

A preparation containing 25,000 IU of urokinase precursor, 20 mg of human albumin and 6.4 mg of table salt (NaCl) was prepared in the same manner as in Example 1.

EXAMPLE 3

50,000 IU of a purified urokinase precursor (specific acitivity: 135,000 IU/mg protein), 40 mg of human albumin and 12.8 mg of sodium citrate were processed in the same manner as in Example 1 to prepare a preparation containing 50,000 IU of urokinase precursor, 40 mg of human albumin and 12.8 mg of sodium citrate.

EXAMPLE 4

25,000 IU of a purified urokinase precursor (specific activity: 135,000 IU/mg protein), 20 mg of human albumin, and 6.4 mg of arginine were dissolved in 1 ml of a 0.1M phosphate buffer (pH, 7.0), aseptically filtered, charged in a vial bottle, and then freeze dried to prepare a prepation containing 25,000 IU of urokinase precursor, 20 mg of human albumin and 6.4 mg of arginine.

EXAMPLE 5

A preparation containing 25,000 IU of urokinase precursor, 20 mg of human albumin and 6.4 mg of sodium glutamate was prepared in the same manner as in Example 1.

EXAMPLE 6

50,000 IU of a purified urokinase precursor (specific activity: 135,000 IU/mg protein), 40 mg of human albumin and 12.8 mg of arginine were processed in the same manner as in Example 1 to prepare a preparation containing 50,000 IU of urokinase precursor, 40 mg of human albumin and 12.8 mg of arginine.

SYNTHESIS EXAMPLE

Cultivated human kidney cells were grown for 3 days in a 0.1% human serum albumin-added non-serum culture, and the fermentation broth thus obtained was subjected to centrifugal separation. The resulting supernatant was frozen and stored. The pooled supernatant was adjusted to pH 5.5 and contacted with CM-Sephadex C-50. After washing a column with a 0.16 M phosphate buffer (pH 5.5), the adsorbed urokinase precursor was eluted with a 0.16 M phosphate buffer (pH, 8.5).

Among hybridomas prepared by fusion using polyethylene glycol of spleen cells of BALB/c mouse which had been immunized with the urokinase precursor and mouse myeloma cells, a clone having a high productivity of antibody for the urokinase precursor was selected. From this fermentation broth of the fused cells, an anti-urokinase monoclonal antibody was recovered. This monoclonal antibody was immobilized on BrCN-activated Sepharose 4B (produced by Pharmacia Co., Ltd.)

This monoclonal antibody column was equilibrated with a 0.1 M phosphate buffer (pH, 7.0) containing 0.4 M NaCl, and then contacted with the eluate containing urokinase precursor. After washing the column with a 0.1 M phosphate buffer (pH, 7.0) containing 0.4 M NaCl, the adsorbed urokinase precursor was eluted with a 0.2 M aqueous glycine-HCl solution (pH: 2.5) containing 0.5 M NaCl. The eluate was neutralized and then was passed through a carrier fixed anti-mouse IgG rabbit IgG and was removed a very small amount of exuded mouse IgG. The solution thus passed was freed from bacteria and filtered, and then freeze dried to obtain a highly purified urokinase precursor having a specific activity of at least 80,000 IU/mg.

This purified product, when measured with SDS polyacrylamide gel electrophoresis, showed one band ascribable to a molecular weight of 50,000.

## Claims

1. A urokinase precursor-containing dry preparation comprising a urokinase percursor as a major component and further, as stabilizers, (a) at least one member selected from sodium chloride, sodium citrate and arginine or salts thereof, and (b) albumin, the amount of sodium chloride, sodium citrate and arginine ranging from 2 to 50 mg/10,000 to 250,000 IU of the urokinase precursor.

2. The preparation as claimed in claim 1, wherein said albumin is added in an amount of from 10 to 60 mg/10,000 to 250,000 IU of the urokinase precursor.

3. The preparation as claimed in claim 1, wherein said albumin is human albumin.

4. The preparation as claimed in claim 1, wherein said albumin is a heat-treated albumin treated at 60°C for about 10 hours.

## Patentansprüche

1. Eine Urokinase-Vorstufe-haltige Trockenpräparation, die eine Urokinase-Vorstufe als Hauptbestandteil und zusätzlich als Stabilisatoren (a) mindestens einen Vertreter, ausgewählt aus Natriumchlorid, Natriumcitrat und Arginin oder Salze davon und (b) Albumin enthält, wobei die Menge des Natriumchlorids, Natriumcitrats und Arginins im Bereich von 2 bis 50 mg/10.000 bis 250.000 IU der Urokinase-Vorstufe liegt.

**2.** Präparation nach Anspruch 1, worin das Albumin in einer Menge von 10 bis 60 mg/10.000 bis 250.000 IU der Urokinase-Vorstufe zugesetzt wird.

**3.** Präparation nach Anspruch 1, worin das Albumin Humanalbumin ist.

**4.** Präparation nach Anspruch 1, worin das Albumin hitzebehandeltes Albumin ist, das bei 60°C ca. 10 Stunden behandelt wurde.

**Revendications**

**1.** Préparation sèche contenant un précurseur d'urokinase comprenant un précurseur d'urokinase comme constituant principal et, en outre, comme stabilisants, (a) au moins un membre sélectionné parmi le chlorure de sodium, le citrate de sodium et l'arginine ou ses sels, et (b) de l'albumine, la quantité de chlorure de sodium, de citrate de sodium et d'arginine allant de 2 à 50 mg/10 000 à 250 000 UI du précurseur d'urokinase.

**2.** Préparation comme revendiquée dans la revendication 1, dans laquelle ladite albumine est ajoutée dans une quantité allant de 10 à 60 mg/10 000 à 250 000 UI du précurseur d'urokinase.

**3.** Préparation comme revendiquée dans la revendication 1, dans laquelle ladite albumine est de l'albumine humaine.

**4.** Préparation comme revendiquée dans la revendication 1, dans laquelle ladite albumine est une albumine traitée par la chaleur, traitée à 60°C pendant environ 10 heures.